# EUROPEAN PATENT APPLICATION

(11) **EP 4 223 213 A1**
(43) Date of publication of application: **09.08.2023**
(21) Application number: 22155295.3
(22) Date of filing: 04.02.2022
(51) Int. Cl.: A61B 5/00, A61B 5/145, B81C 1/00, G02B 6/42

(54) **SENSOR DEVICE**

(71) Applicant: Indigo Diabetes N.V., 9052 Gent (BE)
(72) Inventor: The designation of the inventor has not yet been filed
(74) Representative: Winger

(57) **Abstract**

A sensor device (1) comprising a semiconductor platform (2) with a sensor element (3), a top casing element (51) and a bottom casing element (52). The top casing element (51) is contacting the bottom casing element (52) so as to form a rigid casing (5) defining a cavity (8), and the top casing element (51) furthermore contacting the semiconductor platform (2) so that the semiconductor platform (2) is positioned in the cavity (8) and so that a selected region of the semiconductor platform (2) including the sensor element (3) is contactable by the environment. Furthermore, the top casing element (51), the bottom casing element (52) and the selected region of the semiconductor platform (2) are arranged so that the cavity is hermetically sealed.

## Description

### Technical field of the invention

The present invention relates to the field of sensor devices, More particularly, the present invention relates to a sensor device, such as for example a sensor device for measuring one or more metabolites, like e.g. glucose, in the body of a living creature.

### Background of the invention

Sensor devices are ever more used as an aid in diagnosing or monitoring of illnesses or medical conditions, as well as the quality of products. In particular wearable and implantable sensor devices may enable continuous monitoring and high convenience to the end user. Such sensor devices, especially when implanted subcutaneously, require a high structural integrity. This is not only required to prevent any harm to the user, but also to ensure continuous functioning of the sensor device. In particular, the semiconductor platform that is typically present in sensor devices and that is responsible for electronic and/or photonic data processing, is often sensitive to, i.e., easily damaged by, mechanical stress or humidity.

In sensor devices of the prior art, a top casing is often bonded to e.g. a semiconductor platform. The top casing and the semiconductor platform, in which electronic and/or photonic circuits are provided, may define a cavity that is sealed from the environment, such that the electronic and/or photonic circuits are hermetically separated from the environment. The lifetime of the sensor device, such as that of the semiconductor platform and of the electronic and/or photonic components present in and on the platform, is preferably as long as possible. In particular for implantable sensor devices, a long lifetime is strongly preferred, so that the number of medical procedures required to be applied to the user for implanting and removing the implantable sensor device may be as low as possible. The lifetime of sensor devices of the state of the art may, however, be limited by interactions with the environment, or by mechanical stress exerted on the semiconductor platform.

There is thus still a need in the art for devices and methods that address at least some of the above problems.

### Summary of the invention

It is an object of the present invention to provide good sensing devices. It is further object of the present invention to provide good methods for forming said sensing devices.

The above objective is accomplished by a method and apparatus according to the present invention.

It is an advantage of embodiments of the present invention that a cavity, comprising the semiconductor platform, is defined by a bottom casing element and a top casing element that mechanically, i.e., physically, contact each other. It is an advantage of embodiment of the present invention that mechanical stress exerted on a semiconductor platform present in the cavity of the sensor device may be limited. It is an advantage of embodiments of the present invention that mechanical stress exerted on a rigid casing of the sensor device is substantially not transferred to the semiconductor platform. It is an advantage of embodiments of the present invention that the usable, e.g., hermetic, volume is large. It is an advantage of embodiments of the present invention that a large volume (i.e., the cavity) is available for components of the sensor device, such as a (large) power sources and/or a (large) semiconductor platform, that may be separated from the environment.

In a first aspect, the present invention relates to a sensor device, e.g., an implantable sensor device. The implantable sensor device comprising a semiconductor platform with a sensor element, a top casing element and a bottom casing element. The top casing element is contacting the bottom casing element so as to form a rigid casing defining a cavity, and the top casing element furthermore contacting the semiconductor platform so that the semiconductor platform is positioned in the cavity and so that a selected region of the semiconductor platform including the sensor element is contactable by the environment. Furthermore, the top casing element, the bottom casing element and the selected region of the semiconductor platform are arranged so that the cavity is hermetically sealed.

Whereas the present invention advantageously can be used for implantable devices, embodiments of the present invention are not limited thereto and may in general be used for devices combining external sensing or external contacting with the use of a hermetic cavity.

In embodiments, a first contact area between the bottom casing element and the top casing element of the casing is at least two times large, preferably at least four times as large, more preferably at least ten times as large, as a second contact area between the casing, e.g., the top casing element, and the semiconductor platform. It is an advantage of these embodiments that, due to the large contact area between the bottom and top casing element, and the relatively small contact area between the casing and the semiconductor platform, mechanical coupling between the semiconductor platform and the casing is small compared to mechanical coupling between the top and bottom casing element. As such, when external stress is exerted on, for example, the top casing element of the casing, the stress may be predominantly transferred to the bottom casing element of the casing, not to the semiconductor platform.

In embodiments, a region of a surface of the semiconductor platform, the region circumfering all contact surfaces defining the second contact area, comprises less than 50%, preferably less than 35%, of the area of the surface. The region may have any suitable shape, such as for example a circular shape, a rectangular shape or any other shape. It is an advantage of these embodiments that the contact surfaces defining the second contact area are located close to each other, i.e., are confined to the (e.g., small) region of the semiconductor platform. Thereby, stress-induced deformation of the casing may result in deformation of the semiconductor platform only in said small region of the semiconductor platform. Preferably, stress-sensitive parts of the semiconductor platform are located outside of said region.

In embodiments, the cavity is hermetically separated from an environment of the sensor device. It is an advantage of these embodiments that the semiconductor platform may be protected from the environment, e.g., from fluids and/or chemicals, in the environment. The hermetic separation may comprise that fluids, for example, gas, such as water vapor, and liquid, such as water, and preferably any molecules dissolved therein, are prevented from moving from the environment into the cavity. In embodiments, the hermetic separation may be due to separation by the casing. In different embodiments wherein the sensor element is exposed to the environment through an opening, the hermetic separation may be further due to the sensor element. Instead or in addition, the opening may be blocked by an inert part of the semiconductor platform. In embodiments, the sensor device further comprises sealing material, different from the casing and the sensor element, for the hermetical separation. However, preferably, the hermetic separation is solely due to the casing, and possibly the sensor element exposed to the environment.

In embodiments, the casing being rigid comprises that the casing is formed of a material having a Young's modulus of at least 10 GPa in standard conditions (i.e., at 25°C and 1 atm). In embodiments, the casing is transparent to radiation used for data communication or energy transfer. Examples of frequencies that can be used may be in the range 100 to 200kHz, although embodiments are not limited thereto and also other frequencies can be used. In embodiments, the top casing element and the bottom casing element comprise glass or ceramics. Advantageously, the coefficient of thermal expansion is matching the substrate as good as possible. It is an advantage that glass or ceramics may be inert, and has a high rigidity (the Young's modulus of glass is typically higher than 50). Glass may, furthermore, be transparent to radiation, so that, for example, wireless data transfer or wireless charging of a power supply is possible. It is an advantage that glass is transparent to a broad frequency range, specially in high frequency communication bands such as for example 2.5GHz or 13.56MHz, but not limited to these frequencies, so that, for example, communication between elements present in the cavity and a communication element in the environment is possible without shielding caused by the housing. As far as ceramics are transparent for the radiation used for power or data transfer, also ceramics can advantageously be used.

In embodiments, the casing comprises an opening. The opening may be adapted for exposing the sensor element at least partially to an environment of the sensor device. It is an advantage of these embodiments that, although the semiconductor platform is protected from chemicals and stress from the environment, the sensor element may be exposed to the environment of the sensor device. Thereby, chemical and/or physical interaction between the sensor element and the environment may be possible. This is particularly advantageous when the sensor element is, e.g., a chemical sensor, or an optical sensor using for example evanescent sensing. In embodiments, an area of a cross-section of the opening, in a plane perpendicular to a direction from a first end to a second end of the opening, is less than 30, preferably less than 20%, more preferably less than 10%, of a total area of an exterior surface of the casing.

In embodiments, the sensor element comprises an optical, e.g., a photonic, sensor. That is, the sensor element may comprise a light emitter for emitting light and a light receiver adapted for receiving the light from the light emitter, typically after transmission through or reflection by a material to be analysed. Thereby, the material may be spectrally analysed for determining whether analytes are present in the material, and possibly at what concentration. For example, the material to be analysed may be blood, interstitial fluid, tissue , and the analyte may be one or more of acetate, amino acids, ammonia, creatinine, ethanol, glucose, homocysteine, ketones, lactate, lipids, phenyl sulfate, proponic acid, urea, pyruvate, temperature, vitamin B11 and/or B12, and water, the present invention not being limited thereto. It is an advantage of these embodiments that spectroscopy is a non-invasive detection technique, so that no sample extraction may be required. It is to be noted, as indicated above, that embodiments are not limited to implantable sensors but that for example also other systems may be envisaged such as for example systems for measuring petrochemical components, systems for measuring ethanol in distillation tanks, etc. Furthermore, spectroscopy may be performed in real time, e.g. based on continuously or intermittently measurements, so that trend monitoring of the analytes is possible. Also measuring at predetermined distinct moments in time may be an option. In embodiments, the optical sensor is located in the cavity, and is configured for analysing optical frequencies to which the casing, which is preferably formed of glass, is transparent.

In embodiments, the sensor device comprises a power source, located in the cavity. The power source may enable internal powering of the sensor and the silicon circuit. The casing of the present invention enables the inclusion of a power source, larger than usual, in the cavity. In some embodiments, the power source is separated from at least one of the surfaces of the casing by a spacer, although embodiments are not limited thereto. Having a spacer between the power source and the casing, assists in having a low mechanical coupling between the power source and the casing. Thereby, stress exerted on the casing is substantially not transferred to the power source, i.e., to elements in the cavity. The spacer is preferably formed of a material having a very low Young's modulus of at most 10 MPa. In embodiments, the spacer is formed of e.g. poly-dimethyl siloxane or a similar rubber-like material, but could also be any material, with or without functionality such as polyimide-laminates, FR4 laminates or other. The separation, i.e., a thickness of the flexible spacer, is preferably at least 0.1 mm, such as at least 1 mm.

In embodiments, the power source is separated from the semiconductor platform by a further spacer. Any features of the further flexible spacer may be independently as described with respect to the spacer above. By having the further flexible spacer between the power source and the semiconductor platform, mechanical coupling between the power source and the semiconductor platform may be low. Thereby, stress exerted on the casing and transferred to the power source may be substantially not transferred to the semiconductor platform. In addition, the further flexible spacer may serve to counteract for mechanical stress exerted by the power source, which may, for example, be due to typical swelling of the power source, for example, as a result of chemical processes occurring during charging or use.

In preferred embodiments, at least one side of at least one, preferably of at least two, more preferably of each pair of opposing sides of the semiconductor platform is separated from the casing, the separation comprising a gap filled with air or at least one element, such as for example a flexible circuit board. The pair of opposing sides may, for example, be: a top surface and a bottom surface; a left surface and a right surface; and a front surface and a back surface, of the semiconductor platform. In preferred embodiments, at most two sides, preferably at most one side, e.g., exactly one side, of the semiconductor platform is fixedly coupled to the casing. In embodiments, the at least one flexible element separates the semiconductor platform from the top casing element and/or from the bottom casing element. In embodiments, the semiconductor platform may be fixedly connected with one of the top casing element or the bottom casing element, e.g., at a top surface of the semiconductor platform, and flexibly coupled with another of the top casing element or the bottom casing element, e.g., at a bottom surface of the semiconductor platform. As such, transfer of stress from the casing to the semiconductor platform may be limited. Any features of the flexible element, such as for example a flexible circuit board, may be independently as described with respect to the spacer above. In embodiments, the at least one flexible element may comprise a flexible spacer and/or the further flexible spacer.

In embodiments, the power source is a rechargeable power source. In embodiments, the sensor device comprises an antenna, configured for wireless recharging (also known as wireless charging, remote charging, or inductive charging) of the rechargeable power source. In these embodiments, the casing is preferably transparent to an alternating electric field used for the charging, e.g., 25kHz, 100kHz, 13.56MHz or any other suitable frequency. By enabling wireless recharging of the power source, the sensor device may remain sealed from the environment during the charging. Furthermore, when the sensor device is implanted subcutaneously, the rechargeable power source may be charged by an alternating electric field propagating through the skin, which may improve comfort to the user.

Although the rigid casing is understood to comprise the bottom casing element and the top casing element, this does not limit the present invention to casings comprising a bottom and top casing element that may be, or have been (e.g., before welding) physically separatable from each other. Indeed, as an example, the top and bottom casing elements may form an integral part of the casing. As another example, instead, the rigid casing may be formed by welding a left casing element and a right casing element together: in this example, the left casing element may comprise a first part of each of the top and bottom casing elements, and the right casing may comprise a second part of each of the top and bottom casing elements. In preferred embodiments, the top casing element comprises a substantially plane element, and the bottom casing element comprises a substantially plane element. In these embodiments, either the top casing element or the bottom casing element may comprise, e.g., be integral with, a circumferential element. In these embodiments, the cavity may be substantially defined by the plane elements of the top and bottom casing elements, and by the circumferential element.

The semiconductor platform is typically thin, e.g., having a thickness of less than 1 mm, for example between 300 µm and 800 µm, which may make them sensitive to stress, mechanical impact, and interactions with chemicals in the environment. In embodiments, the semiconductor platform is a silicon-based platform, such as for example a silicon platform or a silicon-nitride based platform. Electronic and/or photonic components, such as an electronic and/or a photonic integrated circuit, may be present in the platform and/or on the platform communicatively coupled to the platform. The sensor element may be communicatively coupled to the semiconductor platform. Thereby, said electronic and/or photonic components may, for example, be for processing of a signal received by the sensor element, or for storing the signal and/or processed signal. In embodiments, the sensor device comprises a receiver configured for receiving communication signals from an external device. In embodiments, the sensor device comprises a transmitter configured for transmitting communication signals to an external device. The receiver and/or transmitter may be configured for communicating via bluetooth, e.g., at from 2.4 to 2.5GHz. Said receiver and/or transmitter may be located in the cavity. Said receiver and/or transmitter may be communicatively coupled to the semiconductor platform. Said electronic and/or photonic components may be configured for generating or processing communication signals transmitted to or received from an external device.

As the semiconductor platform is located in the cavity, the electronic and/or photonic components present in and/or on the platform are separated, hence protected, from an environment of the sensor device. In embodiments, the semiconductor platform comprises a photonic integrated circuit. The photonic integrated circuit may allow for fast and compact optical processing of a signal to be analysed, received by the sensor element, e.g., of optical information received by (a light receiver of) the optical sensor element.

Any features of any embodiment of the first aspect may be independently as correspondingly described for any embodiment of any of the other aspects of the present invention.

In a second aspect, the present invention relates to a method for making a sensor device according to any of the previous claims. The method comprises obtaining a bottom casing element and a top casing element, and a semiconductor platform with a sensor element. The method also comprises contacting the bottom casing element, the top casing element and the semiconductor platform so as to form a rigid casing defining a cavity, so that the semiconductor platform is positioned in the cavity and so that a selected region of the semiconductor platform including the sensor element is contactable by the environment, and so the top casing element, the bottom casing element and the selected region of the semiconductor platform are being arranged so that the cavity is hermetically sealed.

The casing, i.e., the protection, of the sensor device thus comprises both a bottom casing element and a top casing element that physically contact each other. Thereby, external stress exerted on the top casing element is transferred mainly to the bottom casing element - not to the semiconductor platform, as is custom in the field. Thereby, the semiconductor platform is substantially relieved from mechanical responsibility.

The contacting may comprise any technique for connecting, e.g., fastening, the top casing to the bottom casing, such as welding, soldering or gluing. In preferred embodiments, the contacting comprises welding the top casing element to the bottom casing element. The welding typically comprises that part of the top casing element and part of the bottom casing element are merged, e.g. through local material melting, whereafter the molten parts are contacted with each other such to create a hermetic interface. By welding the top casing element to the bottom casing element, for example when the casing elements comprise glass, a rigid bond is achieved between the top and bottom casing element, so that there is good mechanical coupling, and, hence, exchange of mechanical stress, between the top and bottom casing element. The joined interfaces can achieve impermeable properties, e.g. to moisture making it hermetic. It is to be noted that whereas welding is very suitable technique, embodiments are not limited thereto and also other techniques can be used.

In embodiments, the top casing element comprises at least one, preferably at least two, top alignment hole, and the bottom casing element comprises at least one, preferably at least two, bottom alignment hole, and wherein the contacting comprises positioning the at least one top alignment hole over the at least one bottom alignment hole. The alignment holes facilitate alignment of the top and bottom casing element with respect to each other. For example, a rod may be shoved through the bottom and top alignment holes during the connecting for facilitating the alignment. Herein, the rod preferably has an outer diameter substantially the same as an inner diameter of the top and bottom alignment holes.

Any features of any embodiment of the second aspect may be independently as correspondingly described for any embodiment of any of the other aspects of the present invention.

In a third aspect, the present invention relates to a use of the sensor device according to embodiments of the first aspect for detecting one or more analytes. Preferably, the analyte is present in an environment of the sensor device, i.e., not in the cavity of the sensor device. In embodiments, the analyte is a blood, ISF, tissue analyte, such as acetate, amino acids, ammonia, creatinine, ethanol, glucose, homocysteine, ketones, lactate, lipids, phenyl sulfate, proponic acid, urea, pyruvate, temperature, vitamin B11 and/or B12, and water, but not limited thereto.

Any features of any embodiment of the third aspect may be independently as correspondingly described for any embodiment of any of the other aspects of the present invention.

Particular and preferred aspects of the invention are set out in the accompanying independent and dependent claims. Features from the dependent claims may be combined with features of the independent claims and with features of other dependent claims as appropriate and not merely as explicitly set out in the claims.

Although there has been constant improvement, change and evolution of devices in this field, the present concepts are believed to represent substantial new and novel improvements, including departures from prior practices, resulting in the provision of more efficient, stable and reliable devices of this nature.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### Brief description of the drawings

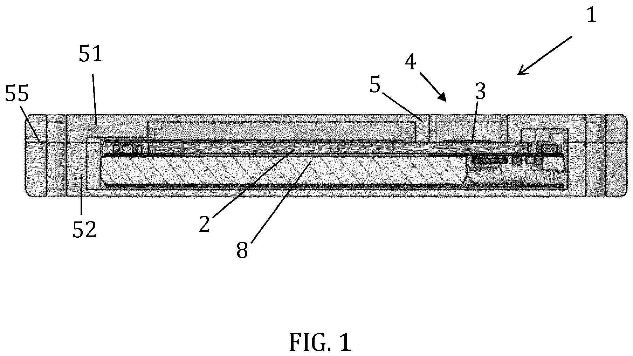

FIG. 1 is a schematic vertical cross-section of a sensor device according to embodiments of the present invention.

### Description of illustrative embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second, third and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. The term "comprising" therefore covers the situation where only the stated features are present and the situation where these features and one or more other features are present. The word "comprising" according to the invention therefore also includes as one embodiment that no further components are present. Thus, the scope of the expression "a device comprising means A and B" should not be interpreted as being limited to devices consisting only of components A and B. It means that with respect to the present invention, the only relevant components of the device are A and B.

Similarly, it is to be noticed that the term "coupled", also used in the claims, should not be interpreted as being restricted to direct connections only. The terms "coupled" and "connected", along with their derivatives, may be used. It should be understood that these terms are not intended as synonyms for each other. Thus, the scope of the expression "a device A coupled to a device B" should not be limited to devices or systems wherein an output of device A is directly connected to an input of device B. It means that there exists a path between an output of A and an input of B which may be a path including other devices or means. "Coupled" may mean that two or more elements are either in direct physical or electrical contact, or that two or more elements are not in direct contact with each other but yet still co-operate or interact with each other.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description provided herein, numerous specific details are set forth. However, it is understood that embodiments of the invention may be practiced without these specific details. In other instances, well-known methods, structures and techniques have not been shown in detail in order not to obscure an understanding of this description.

The invention will now be described by a detailed description of several embodiments of the invention. It is clear that other embodiments of the invention can be configured according to the knowledge of persons skilled in the art without departing from the technical teaching of the invention, the invention being limited only by the terms of the appended claims.

In a first aspect, the present invention relates to a sensor device comprising a semiconductor platform comprising a sensor element, a top casing element and a bottom casing element. The top casing element is contacting the bottom casing element so as to form a rigid casing defining a cavity. The top casing element furthermore is contacting the semiconductor platform so that the semiconductor platform is positioned in the cavity and so that a selected region of the semiconductor platform including the sensor element is contactable by the environment. The top casing element, the bottom casing element and the selected region of the semiconductor platform are being arranged so that the cavity is hermetically sealed.

In a second aspect, the present invention relates to a method for making a sensor device according to the first aspect. The method comprises obtaining a bottom casing element and a top casing element, and a semiconductor platform with a sensor element. The method also comprises contacting the bottom casing element, the top casing element and the semiconductor platform so as to form a rigid casing defining a cavity, so that the semiconductor platform is positioned in the cavity and so that a selected region of the semiconductor platform including the sensor element is contactable by the environment. The top casing element, the bottom casing element and the selected region of the semiconductor platform thereby are being arranged so that the cavity is hermetically sealed. The top casing element and the bottom casing element may be contacted to each other. The top casing element and the semiconductor platform may be contacted to each other. The contacting may thus result in creating the hermetically sealed cavity.

Reference is made to FIG. 1, which is a schematic representation of an example of a sensor device 1 in accordance with embodiments of the present invention, wherein, however, for reasons of clarity, some elements are not shown. In this example, a semiconductor platform 2, e.g., a photonic substrate 2, which may be made, for example, of silicon, silicon-oxide, silicon-carbide, or silicon-nitride or other typical materials of the semiconductor industry. A sensor element 3 is located on the semiconductor platform 2. In this example, the sensor element 3 comprises a light emitter for emitting light and a light receiver adapted for receiving the light from the light emitter, typically after transmission through or reflection by a material to be analysed. In this example, the sensor device further comprises an electronic circuit on the semiconductor platform. The sensor device of the present example further comprises a power source holder comprising a power source. The power source holder of the present example further comprises battery holder feet that may be connected, e.g., glued, to the casing, e.g., to the top casing element (not shown). Thereby, there may be mechanical decoupling between the power source and the semiconductor platform 2, preventing transfer of mechanical stress. An electrical input and/or output of the power source may be electrically connected to an electrical output and/or input, respectively, of the electronic circuit. Herein, the respective inputs and outputs may be soldered together to form a robust electrical connection.

In the present example, embodiments of the present invention not being limited thereto, the casing 5, consisting of a top casing element 51 and a bottom casing element 52, is shown. In this example, the casing 5 consists of glass, and the top casing element 51 and the bottom casing element 52 are connected so as to form a cavity 8. There is a large first contact area 55 between the top casing element 51 and the bottom casing element 52. The top casing element 51 and the bottom casing element 52 are, in this example, rigidly connected to each other by welding. The contact area 55 in the present example are welded interfaces providing hermetical sealing of the hermetic cavity 8.

The top casing element 51 in some embodiments may comprise alignment holes extending from a top surface to a bottom surface of the top casing element 51, and the bottom casing element 52 may comprise alignment holes extending from a top surface to a bottom surface of the bottom casing element 52. Before the connecting and welding, through each of the top alignment holes, a rod may be positioned. Next, the rod may be positioned through one of the, i.e., the respective, bottom alignment holes of the bottom casing element 52. This may result in good alignment of the top 51 and bottom casing element 52, so that a cavity 8 may be formed that is preferably hermetically separated from an environment.

The cavity 8 is defined by the casing 5. In this example, the top casing element 51 comprises an opening for exposing a sensor element 3. In this example, a small, inert (with respect to the environment), part of a top surface of the semiconductor platform 2, exposed to the environment, thus defining the exposed external area 4, blocks the opening, ensuring that the cavity 8 is hermetically separated, i.e., sealed, from the environment. The sensor element 3 is, in this example, a chemical sensor element, for sensing of analytes in the environment by interaction of said analytes with the sensor element 3. However, the invention is not limited thereto, and instead, the sensor element 3 may be an optical, e.g., spectroscopic, sensor element 3, or a physical sensor element 3, e.g., for sensing a pressure or temperature. In this example, to enable the formation of a cavity 8 that is hermetically separated from the environment, there is a, small, second contact area between the top casing element 51 and the semiconductor platform 2. Herein, the first contact area 55 is at least 10 times as large as the second contact area, so that stress is more efficiently transferred between the top 51 and bottom casing element 52 than between the casing 5 and the semiconductor platform 2. Furthermore, although the connection between the semiconductor platform 2 and the top casing element 51 is rigid (e.g., formed by welding, the semiconductor platform 2 is, for the rest, substantially free to move inside the cavity (wherein movement may be, for instance, induced by stress-related deformation of the top casing element 51). Indeed, a further flexible element is present between the element below the semiconductor platform 2 - in this example, the power source - and the semiconductor platform 2. In addition, a flexible element is present between the element below the semiconductor platform 2 - in this example, the power source - and the bottom casing element. As such, the semiconductor platform 2 is fixedly connected with the top casing element 51 at a top surface of the semiconductor platform 2, and at the same time the semiconductor platform 2 is flexibly coupled to the bottom casing element 52 at a bottom surface of the semiconductor platform 2. In other words, a gap between the semiconductor platform 2 and the bottom casing element 52 comprises a flexible spacer, which in this example consists of the further flexible spacer and the flexible spacer. While the fixed connection enables structural integrity of the sensor device 1, the flexible coupling promotes inefficient transfer of stress between the casing 5 and the semiconductor platform 2.

In this example, an electrical connector is used for electrically coupling of the power source and the electronic circuit. The electrical connector is located adjacent the semiconductor platform 2, in a direction perpendicular to a direction from the electrical connector to the power source. Thereby, the presence of the electrical connector does not impact the overall thickness of the sensor device 1. The electrical connector may provide a more robust and reliable electrical coupling, and may require an easier assembly of the sensor device 1, than when, instead, soldering or gluing is used to establish the electrical coupling. Herein, a flexible sheet is present between the electrical connector and the power source so as to further mechanically decouple the electrical connector from the power source. In the example, the power source is a rechargeable power source. The power source is connected to an antenna, configured for wireless recharging of the rechargeable power source. The battery holder feet contact the casing 5, e.g., may be fixated to the casing with glue, so that the power source may not move closer to the semiconductor platform 2.

In this example, at least one side of each pair of opposing sides of the semiconductor platform 2 is separated from the casing 5, the separation comprising a gap filled with air or at least one flexible element, wherein said separation is in a direction normal to the side, i.e., perpendicular to the side in a direction away from the side. That is, first pair of opposing sides is a top surface and a bottom surface of the semiconductor platform 2. Herein, the bottom surface is separated from the casing 5, i.e., from the bottom casing element 52. Said separation comprises two gaps filled a flexible element, that is, the further flexible spacer and the flexible spacer. A second pair of opposing sides is the left side and the right side. In this example, both sides and are separated from the casing 5, wherein the separation comprises a gap filled with air. A third pair of sides is the front and back side (not shown), both being separated from the casing 5, wherein the separation comprises a gap filled with air. In this example, only a single side, i.e., the top side of the semiconductor element 2 is fixedly coupled to the casing 5. This ensures a large freedom to move by the semiconductor element 2 in response to stress exerted by the casing 5, without being hampered by further fixed connections to the casing 5.

Whereas in the example, a rectangular shaped region was used, it is to be noted that embodiments of the present invention are not limited thereto, and for example circular shaped regions can also be used, aside from other shapes. It is an advantage of embodiments that the contact surfaces defining the contact area between the casing 5 and the semiconductor platform 2 are located close to each other, i.e., are confined to a small area of the semiconductor platform 2. Thereby, stress-induced deformation of the casing may result in deformation of the semiconductor platform 2 only in the small area of the semiconductor platform. Preferably, stress-sensitive parts of the semiconductor platform 2 are substantially located outside of the small area, so that they are less subjected to stress exerted by the casing 5 on the semiconductor platform 2. In this example, the small area is approximately 4%, i.e. for example less than 10%, of a total area of an exterior surface 50 of the casing 5.

It is to be understood that although preferred embodiments, specific constructions and configurations, as well as materials, have been discussed herein for devices according to the present invention, various changes or modifications in form and detail may be made without departing from the scope of this invention. Steps may be added or deleted to methods described within the scope of the present invention.

## Claims

1. An sensor device (1) comprising:
- a semiconductor platform (2) comprising a sensor element (3),
- a top casing element (51),
- a bottom casing element (52),
the top casing element (51) contacting the bottom casing element (52) so as to form a rigid casing (5) defining a cavity (8),
the top casing element (51) furthermore contacting the semiconductor platform (2) so that the semiconductor platform (2) is positioned in the cavity (8) and so that a selected region of the semiconductor platform (2) including the sensor element (3) is contactable by the environment,
the top casing element (51), the bottom casing element (52) and the selected region of the semiconductor platform (2) being arranged so that the cavity is hermetically sealed.

2. The sensor device (1) according to claim 1, wherein a first contact area (55) between the bottom casing element (52) and the top casing element (51) of the casing (5) is at least two times as large, preferably at least four times as large, as a second contact area between the top casing element (51) and the semiconductor platform (2).

3. The sensor device (1) according to claim 2, wherein a region of a surface of the semiconductor platform, the region circumfering all contact surfaces defining the second contact area, comprises less than 50%, preferably less than 35%, of the area of the surface.

4. The sensor device (1) according to any of the previous claims, wherein the top casing element (51) and the bottom casing element (52) comprises any of ceramics or glass.

5. The sensor device (1) according to any of the previous claims, wherein the casing (5) comprises an opening thus providing an exposed external area (4), adapted for exposing the sensor element (3) at least partially to an exterior of the sensor device (1).

6. The sensor device (1) according to any of the previous claims, wherein the sensor element (3) is a photonic sensor and/or wherein the sensor device (1) comprises a power source, located in the cavity (8).

7. The sensor device (1) according to claim 6, wherein the power source is separated from at least one of the surfaces of the casing (5) by a spacer.

8. The sensor device (1) according to claim 6 or 7, wherein the power source is separated from the semiconductor platform (2) by a further flexible spacer.

9. The sensor device (1) according to any of claims 6 to 8, wherein the power source is a rechargeable power source, and wherein the sensor device (1) comprises an antenna, configured for wireless recharging of the rechargeable power source.

10. The sensor device (1) according to any of the previous claims, wherein at least one side of at least one, preferably of at least two, more preferably of each, pair of opposing sides of the semiconductor platform (2) is separated from the casing (5), the separation comprising a gap filled with air or at least one flexible element.

11. The sensor device (1) according to any of the previous claims, wherein the semiconductor platform (2) comprises a photonic integrated circuit.

12. A method for making a sensor device (1) according to any of the previous claims, the method comprising:
obtaining a bottom casing element (52) and a top casing element (51), and a semiconductor platform (2) with a sensor element (3), and
contacting the bottom casing element (52), the top casing element (51) and the semiconductor platform (2)so as to form a rigid casing (5) defining a cavity (8), so that the semiconductor platform (2) is positioned in the cavity (8) and so that a selected region of the semiconductor platform (2) including the sensor element (3) is contactable by the environment,
the top casing element (51), the bottom casing element (52) and the selected region of the semiconductor platform (2) being arranged so that the cavity is hermetically sealed.

13. The method according to claim 12, wherein the contacting comprises one or more of laser-welding, torch-welding, arc-welding, diffusion bonding, recrystallisation bonding, anodic bonding, thermocompression bonding, glueing or soldering the top casing element (51) to the bottom casing element (52).

14. The method according to any of claims 12 or 13, wherein contacting the bottom casing element, the top casing element and the semiconductor platform comprises contacting the top casing element and the bottom casing element to each other and contacting the top casing element and the semiconductor platform, thus creating the hermetically sealed cavity.

15. The method according to any of claims 12 to 14, wherein the top casing element (51) comprises at least one top alignment hole, and the bottom casing element (52) comprises at least one bottom alignment hole, and wherein the contacting comprises positioning the at least one top alignment hole over the at least one bottom alignment hole.
